# EUROPEAN PATENT APPLICATION

(11) **EP 2 706 123 A1**
(43) Date of publication of application: **12.03.2014**
(21) Application number: 12183211.7
(22) Date of filing: 05.09.2012
(51) Int. Cl.: C12Q 1/68

(54) **In vitro method for determining genotoxic and non-genotoxic carcinogenicity of a compound.**

(71) Applicant: Rijksinstituut Voor Volksgezondheid En Milieu, 3721 MA Bilthoven (NL); Academisch Ziekenhuis Leiden HODN Leids Universitair Medisch Centrum, 2333 ZA Leiden (NL)
(72) Inventor: Melis, Joost Petrus Maria, 2300 RC LEIDEN (NL); Luijten, Mirjam, 3514 TP UTRECHT (NL); Pronk, Theresia Elizabeth, 3994 XR HOUTEN (NL); Steeg, VAN, Harmen, 1261 PG BLARICUM (NL); Mullenders, Leonardus Helena Fredericus, 2300 RC LEIDEN (NL)
(74) Representative: Habets, Winand

(57) **Abstract**

The invention is in the field of molecular diagnostics. More in particular it provides marker genes for determining the genotoxicity and carcinogenicity of compounds. A method according to the invention employs samples obtained from animals exposed to a potentially genotoxic and/or carcinogenic compound and determines expression levels of a number of marker genes in order to distinguish between carcinogenic compounds and non-carcinogenic compounds and between genotoxic and non-genotoxic carcinogenic compounds. Even more in particular, the invention provides an *in vitro* method for determining whether it is likely or unlikely that a compound is a genotoxic carcinogen wherein the expression level of at least one marker gene is determined in a sample obtained from a rodent previously exposed to the compound, wherein the at least one marker gene is selected from the group consisting of *LOC75771, Nup62, Zbtb16* and *Anks4b,* and wherein it is concluded that the compound is likely to be a genotoxic carcinogen if the expression level of *LOC75771* or *Anks4b* is above a predetermined reference value or if the expression level of *Nup62* or *Zbtb16* is below a predetermined reference value or concluding that the compound is unlikely a genotoxic carcinogen if the expression level of *LOC75771* or *Anks4b* is at or below a predetermined reference value or if the expression level of *Nup62* or *Zbtb16* is at or above a predetermined reference value.

## Description

### Field of the invention

The invention is in the field of molecular diagnostics. More in particular it provides marker genes for determining the genotoxicity and carcinogenicity of compounds. A method according to the invention employs samples obtained from animals exposed to a potentially genotoxic and/or carcinogenic compound and determines expression levels of a number of marker genes in order to distinguish between carcinogenic compounds and non-carcinogenic compounds and between genotoxic and non-genotoxic carcinogenic compounds.

### Background of the invention

An accurate assessment of the carcinogenic potential of chemicals and pharmaceutical drugs is essential to protect humans and the environment. Substances are extensively tested before being marketed to the public. Following European legislation, a substance is suspected to be carcinogenic based on genotoxicity assays. Positive genotoxic (GTX) assay results warrant testing in carcinogenicity rodent bioassays. These bioassays are time consuming, expensive, ethically aggravating with respect to animal use and welfare, and result in high percentages of false positives. Additionally, non-genotoxic carcinogens (NGTXC) are not identified using this strategy. Alternatives are therefore highly desirable.

Another disadvantage in carcinogenicity testing under REACH is the fact that non-genotoxic carcinogens are misclassified as a non-carcinogen since the test strategy is based on genotoxic potential of chemicals. A suitable test for identifying these non-genotoxic carcinogens is necessary to protect society from harmful exposure.

For assessment of genotoxic potential of substances, the standard approach is a tiered test strategy including short-term, *in vitro* and *in vivo* genotoxicity tests. A positive *in vitro* genotoxicity result usually triggers further *in vivo* testing. Non-genotoxic carcinogens are currently identified in a 2-year carcinogenicity bioassay. Under REACH, however, the 2-year bioassay is only performed for substances with positive in vivo genotoxicity results. Given that non-genotoxic carcinogens are negative in *(in vitrolin vivo)* genotoxicity tests, this class of carcinogens goes largely undetected.

The 2-year bioassay requires large numbers of animals, the assays are slow, insensitive, and expensive. On top of this, there is considerable scientific doubt on the reliability of the assay, since too many false positive results (so-called rodent carcinogens) are observed.

### Summary of the invention

We have identified several marker genes that may assist in determining the likelihood that a compound is carcinogenic. In a method according to the invention, animals are exposed to the compound for only a short period of time and marker gene expression is determined.

This approach, in contrast to *in vitro* or *in silico* assays, assures relevant route of exposure and full functionality of tissue- and cell-specific responses upon exposure, such as potent biotransformation. In a study employing 5 genotoxic carcinogenic (GTXC) compounds, 7 non-genotoxic carcinogenic (NGTXC) compounds, and 5 non-carcinogenic (NC) compounds we show that GTXC compounds may be distinguished from NGTXC and NC compounds using a panel of 4 marker genes and that NGTXC compounds may be distinguished from NC compounds using a panel of 2 additional marker genes.

In that way, using a two-step assay, carcinogenic compounds may be distinguished from non-carcinogenic compounds by first distinguishing GTXC compounds from NGTXC plus NC compounds and then in a second step distinguishing the NGTXC compounds from the NC compounds.

Hence, the invention relates to an *in vitro* method for determining whether it is likely or unlikely that a compound is a genotoxic carcinogen (GTXC) wherein the expression level of at least one marker gene is determined in a sample obtained from a rodent previously exposed to the compound, wherein the at least one marker gene is selected from the group consisting of *LOC75771, Nup62, Zbtb16* and *Anks4b,* and wherein it is concluded that the compound is likely a genotoxic carcinogen if the expression level of *LOC75771* or *Anks4b* is above a predetermined reference value or if the expression level of *Nup62* or *Zbtb16* is below a predetermined reference value or concluding that the compound is unlikely a genotoxic carcinogen if the expression level of *LOC75771* or *Anks4b* is at or below a predetermined reference value or if the expression level of *Nup62* or *Zbtb16* is at or above a predetermined reference value.

The invention also relates to a method for determining whether it is likely or unlikely that a compound is a carcinogen by first performing a method as described above and concluding that a compound is unlikely to be a genotoxic carcinogen, followed by a step wherein the expression level of at least one second marker gene is determined in a sample obtained from a rodent previously exposed to the compound, wherein the at least one second marker gene is selected from the group consisting of *9030619P08Rik* and *Cyp17a1,* and wherein it is concluded that the compound is likely a non-carcinogenic compound if the expression level of *9030619P08Rik* and/or *Cyp17a1* is below a predetermined reference value or wherein it is concluded that the compound is likely a non-genotoxic carcinogenic compound if the expression level of *9030619P08Rik* and/or *Cyp17a1* is at or above a predetermined reference value.

### Detailed description of the invention

The invention relates to *in vitro* methods for distinguishing between three groups of compounds. In a study employing 5 genotoxic carcinogenic (GTXC) compounds, 7 non-genotoxic carcinogenic (NGTXC) compounds, and 5 non-carcinogenic (NC) compounds we show that GTXC compounds may be distinguished from NGTXC and NC compounds using a panel of 4 marker genes and that NGTXC compounds may be distinguished from NC compounds using a panel of 2 additional marker genes.

The genes used herein are identified by their Entrez-ID, their gene name and their gene symbols as shown in Table 1.

**Table 1**

| **Entrez ID** | **Gene Symbol** | **Gene Name** |
|---|---|---|
| 75771 | *LOC75771* | uncharacterized LOC75771 |
| 72074 | *Anks4b* | ankyrin repeat and sterile alpha motif domain containing 4B |
| 18226 | *Nup62* | nucleoporin 62 |
| 235320 | *Zbtb16* | zinc finger and BTB domain containing 16 |
| | | |
| 13074 | *Cyp17a1* | cytochrome P450, family 17, subfamily a, polypeptide 1 |
| 105892 | *9030619P08Rik* | RIKEN cDNA 9030619P08 gene |

In the present invention, rodents may be exposed to potentially carcinogenic compounds and their tissue may be tested for aberrant gene expression using the marker genes as identified herein. Hence, the invention relates to an *in vitro* method for determining whether it is likely or unlikely that a compound is a genotoxic carcinogen (GTXC) wherein the expression level of at least one marker gene is determined in a sample obtained from a rodent previously exposed to the compound, wherein the at least one marker gene is selected from the group consisting of LOC75771, *Nup62, Zbtb16* and *Anks4b,* and wherein it is concluded that the compound is likely to be a genotoxic carcinogen if the expression level of LOC75771 or *Anks4b* is above a predetermined reference value or if the expression level of Nup62 or Zbtb16 is below a predetermined reference value or concluding that the compound is unlikely to be a genotoxic carcinogen if the expression level of LOC75771 or *Anks4b* is at or below a predetermined reference value or if the expression level of *Nup62* or *Zbtb16* is at or above a predetermined reference value.

In the context of the present invention, the term "exposed" means "contacted" in the broadest sense of the word. Exposure means physically contacting the animal with the compound such as for instance feeding the compound to the animal, injecting the animal with the compound or contacting the animal with the compound by topical exposure. It is also conceivable that the animal is contacted with a volatile compound by inhalation of the compound. It is important that the animal is exposed to the compound at sub-cytotoxic levels and dosages.

The term "tissue" is to be interpreted as any tissue of the animal that contains nucleated cells, such as but not limited to liver, blood, spleen, kidney, bladder, bone marrow, lung, stomach and thymus. The liver is the preferred organ for testing carcinogenicity since it delivers the most reliable results in the shortest time, other tissue or organ are however equally suited.

The method according to the invention may be performed with any kind of rodent, for practical purposes mice or rats are preferred. Laboratory mouse strains, such as inbred strains are most suitable for the purpose in view of costs and reproducibility. By way of a non-limiting example we show herein the method according to the invention performed on C57BL/6J mice. However, C57 Black mice, in particular C57BLC6 mice are equally suited.

The term "Gene Expression Ratio" or "Ratio" as used herein refers to a number describing how much a quantity changes going from an initial to a final value. For example, an initial value of 30 and a final value of 60 corresponds to a Ratio of 2, or in common terms, a two-fold increase. The Gene Expression Ratio is calculated simply as the ratio of the final value to the initial value, i.e. the control expression value. If the initial value is A and final value is B, the Ratio is B/A. As another example, a change from 80 to 20 would be a Ratio of 0.25, while a change from 20 to 80 would be a Ratio of 4. Some practitioners replace a Ratio that is less than 1 by the negative of its inverse, e.g. a change from 80 to 20 would be a fold change of -4 (or in common terms, a four-fold decrease). This is referred herein as "Fold Change". In the tables presented herein we show the Fold Changes of overexpressed genes as values above 1.0 and underexpression as the negative inverse of the Ratio, i.e. values below -1.0. In the figures accompanying this document, the Ratio are shown, i.e. the Ratio of overexpressed genes are shown as values above 1.0, the Ratio of underexpressed genes are shown as values between 0 and 1.

Under the appropriate conditions, a method according to the invention may be performed after exposing the rodent to the compound for a very short period of time. Exposure times of one day, 3 days and 7 days were found to be feasible. In the experimental section, examples are shown wherein the animals were exposed to the compounds for one week. Thereby the exposure time of the animal to the compound is reduced from 2 years to only one week which is a giant leap forward in costs, efforts and animal welfare. Longer exposure times are envisaged when other organs than the liver are used for determining the expression levels. It may be necessary in that case to expose the animal to the compound for 2 weeks or more, such as 3, 4, 5, 6, 7, 8, 9, 10 weeks or more such as 15 or 20 weeks. It may also be feasible to shorten the period of time depending on the choice of the particular marker gene and the sensitivity and reproducibility of the assay used for the detection of the expression levels.

Moreover, the number of experimental animals used may be reduced from 50 to a single mouse. It is preferred however to perform several repeat experiments in order to improve the reliability of the method. In case the method is performed on multiple animals, average expression levels for each gene are to be used instead of individual expression levels. So the reliability and reproducibility of the method improves when the method is performed with 2, 3, 4, 5, 6, 7, 8, 9, or even 10 or more, such as 15, 20, or more individual animals.

Gene expression levels may be determined with every method known in the art for detecting nucleic acid expression levels. Methods such as quantitative PCR, quantitative NASBA, gene sequencing such as next-generation sequencing, RNA sequencing, reporter gene assays or micro-array analysis are particularly preferred. Gene expression levels may also be determined by examining protein expressed by the marker genes disclosed herein, for instance by ELISA techniques.

The expression levels are to be compared to a reference value, also often referred to as cut-off value. Such a reference value may be empirically determined or arbitrarily chosen in order to achieve appropriate specificity and/or sensitivity of the method. A skilled person is fully aware how to choose an appropriate reference value. Such a reference value may advantageously be based on a control expression value.

The control expression value is defined herein as the expression value for a particular gene obtained with a control exposed animal instead of an animal exposed to a carcinogenic or non-carcinogenic compounds. A skilled person is fully aware of ways to determine a control expression value for a particular gene. This may be done using control animals exposed to identical conditions as the test animals with the exception that the control animal is not exposed to the compound to be tested for carcinogenicity and/or genotoxicity. Control expression values may also be obtained using a standard gene expression in a quantitative PCR assay. Such a standard gene is then advantageously a household gene.

The control expression value is used to calculate the Ratio of the gene expression as detailed herein.

The skilled person is also aware of the fact that the number of control animals used for determining the control expression value is at least one but advantageously multiple control animals are used to determine a control expression value. The reliability of the method according to the invention improves when multiple control animals are used to determine the control expression value.

In the experimental section we describe how we exposed C57BL/6J mice for 7 days to a potentially carcinogenic compound and then determined the expression levels of genes *LOC75771, Nup62, Zbtb16* and *Anks4b* in their liver. The results of these experiments are shown in the below tables. Table 2 shows the average Fold Changes of 3 - 4 independent repeat experiments wherein the expression levels of the marker genes *LOC75771, Nup62, Zbtb16* and *Anks4b* are determined under the influence of GTXC compounds. Table 3 shows the average Fold Changes of the expression levels of these genes under the influence of NGTXC compounds and Table 4 shows the average Fold Changes of the expression levels of these marker genes under the influence of NC compounds.

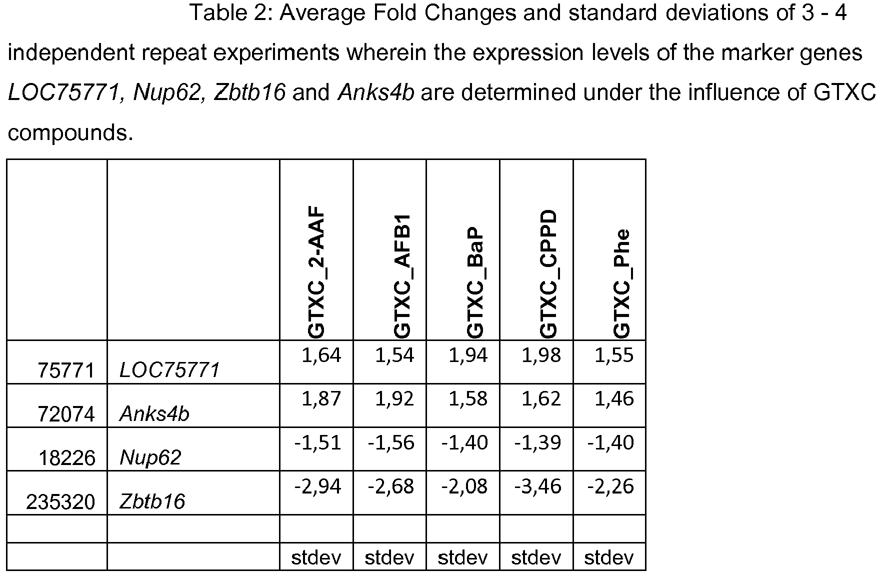

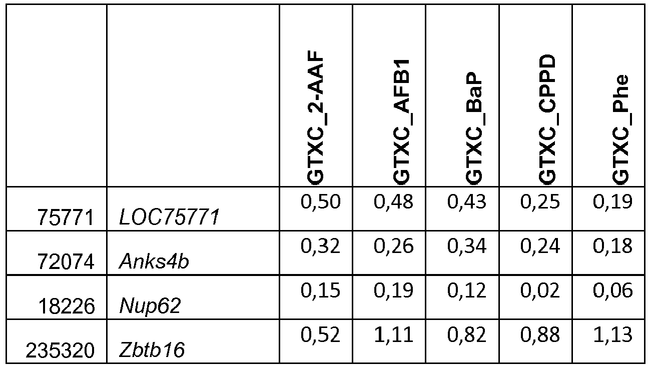

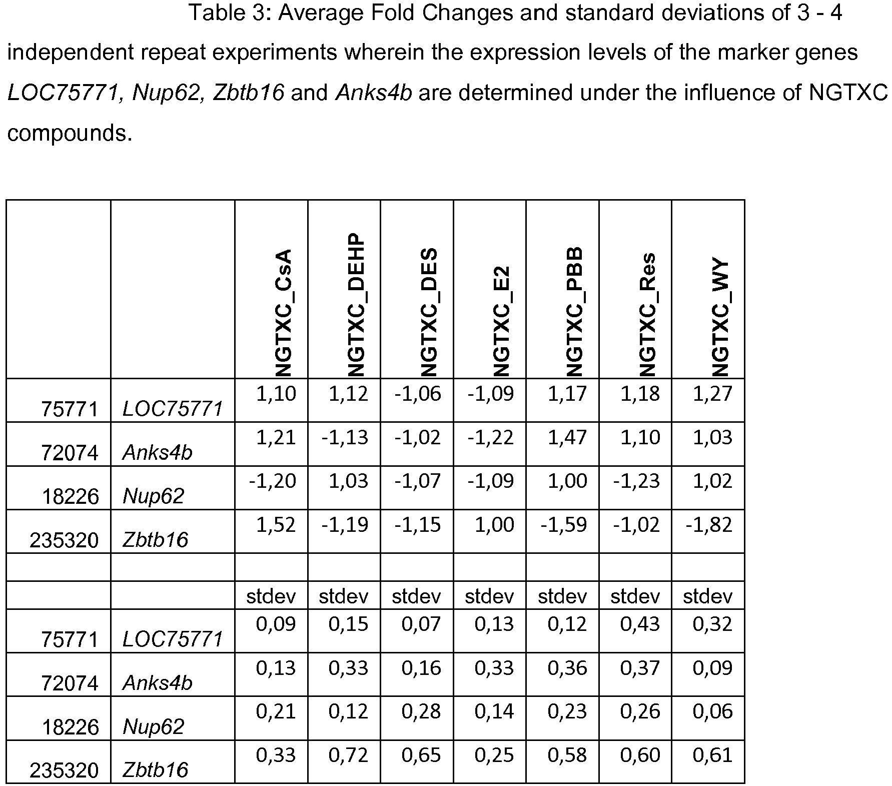

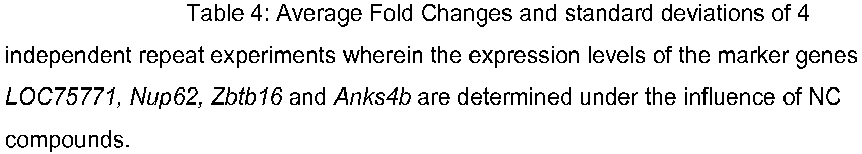

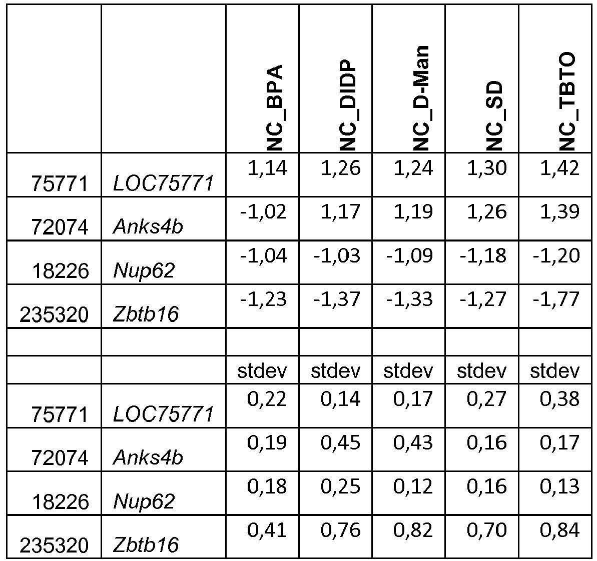

It may be determined from these results that a GTXC compound may be distinguished from a NGTXC or a NC compound on the basis of the Fold Change of expression of marker gene *LOC75771.* In the set-up as chosen, wherein the method was performed in 3 or 4-fold, it is most advantageous to select a predetermined reference value of 1.5 times the control expression value. Under those conditions, all GTXC compounds were found positive in the method according to the invention whereas all NGTXC and NC compounds were negative. This results in a 100% sensitivity and specificity.

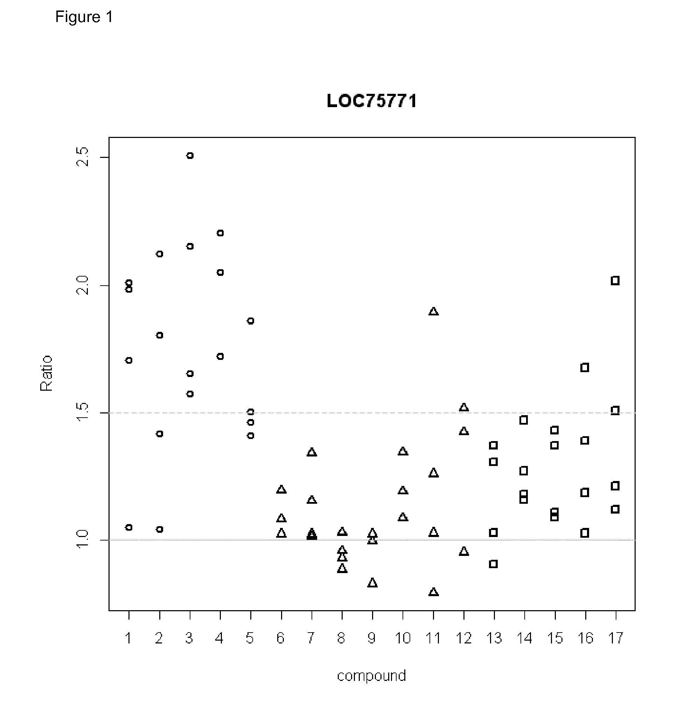

In case the method is performed with a single rodent for each compound, the results are still much better than with any prior art method available. Figure 1 shows that from the 19 individual animals exposed to 5 different GTXC compounds, the method employing marker gene *LOC75771* would have predicted the GTXC compound right in 14/19 cases, i.e. a false negative rate of 5/19 or 26%. Even more importantly, the NGTXC compounds would have been predicted right in 24 out of 26 cases, or a false positive rate of only 2/26 or 8%. The NC compounds would have been predicted right in 17 out of 20 cases or a false positive rate of only 15%.

A skilled person will know how to alter the predetermined reference value in order to obtain the desired specificity and sensitivity of the method.

It may also be determined from these results that a GTXC compound may be distinguished from a NGTXC or a NC compound on the basis of the Fold Change of expression of marker gene *Anks4b.* In the set-up as chosen, wherein the method was performed in 3 or 4-fold, it is most advantageous to select a predetermined reference value of 1.50 times the control expression value. Under those conditions, 4 out of 5 GTXC compounds were found positive in the method according to the invention whereas all NGTXC and NC compounds were negative. This results in a sensitivity of 80% and 100% specificity.

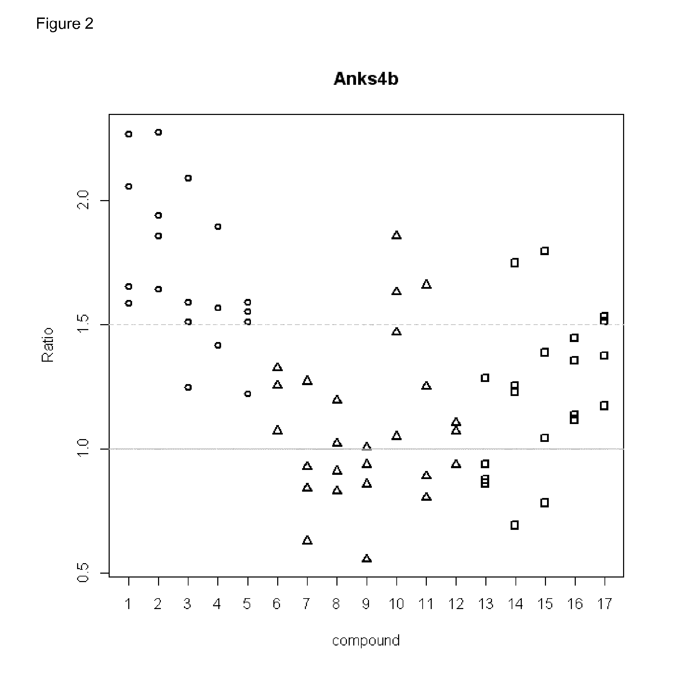

In case the method is performed with a single rodent for each compound, the results are still much better than with any prior art method available. Figure 2 shows that from the 19 individual animals exposed to 5 different GTXC compounds, the method employing marker gene *Anks4b* would have predicted the GTXC compound right in 16/19 cases, i.e. a false negative rate of 3/19 or 16%. Even more importantly, the NGTXC compounds would have been predicted right in 23 out of 26 cases, or a false positive rate of only 3/26 or 12%. The NC compounds would have been predicted right in 16 out of 20 cases or a false positive rate of only 20%.

It may also be determined from these results that a GTXC compound may be distinguished from a NGTXC or a NC compound on the basis of the Fold Change of expression of marker gene *Nup62.* In the experimental set-up as chosen, wherein the method was performed in 3 or 4-fold, it is most advantageous to select a predetermined reference value of -1.33 or 0.75 (1/1,33). Under those conditions, 5 out of 5 GTXC compounds were found positive in the method according to the invention whereas all NGTXC and NC compounds were negative. This results in a sensitivity of 100% and a specificity of 100%.

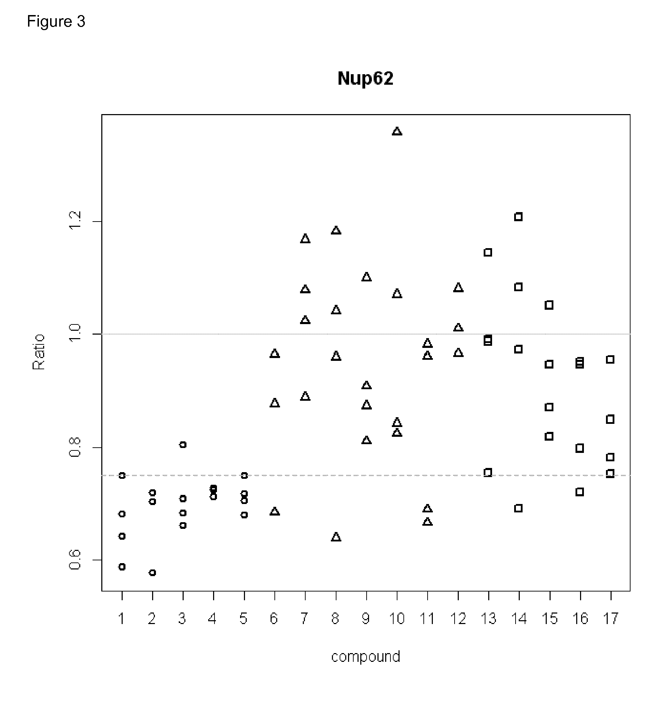

In case the method is performed with a single rodent for each compound, the results are still much better than with any prior art method available. Figure 3 shows that from the 19 individual animals exposed to 5 different GTXC compounds, the method employing marker gene *Nup62* would have predicted the GTXC compound right in 18/19 cases, i.e. a false negative rate of 1/19 or 5%. Even more importantly, the NGTXC compounds would have been predicted right in 22 out of 26 cases, or a false positive rate of only 4/26 or 15%. The NC compounds would have been predicted right in 18 out of 20 cases or a false positive rate of only 10%.

It may also be determined from these results that a GTXC compound may be distinguished from a NGTXC or a NC compound on the basis of the Fold Change of expression of marker gene *Zbtb16.* In the experimental set-up as chosen, wherein the method was performed in 3 or 4-fold, it is most advantageous to select a predetermined reference value of -2.0 or 0.50 (1/2). Under those conditions, 5 out of 5 GTXC compounds were found positive in the method according to the invention whereas all NGTXC and NC compounds were negative. This results in a sensitivity of 100% and a specificity of 100%.

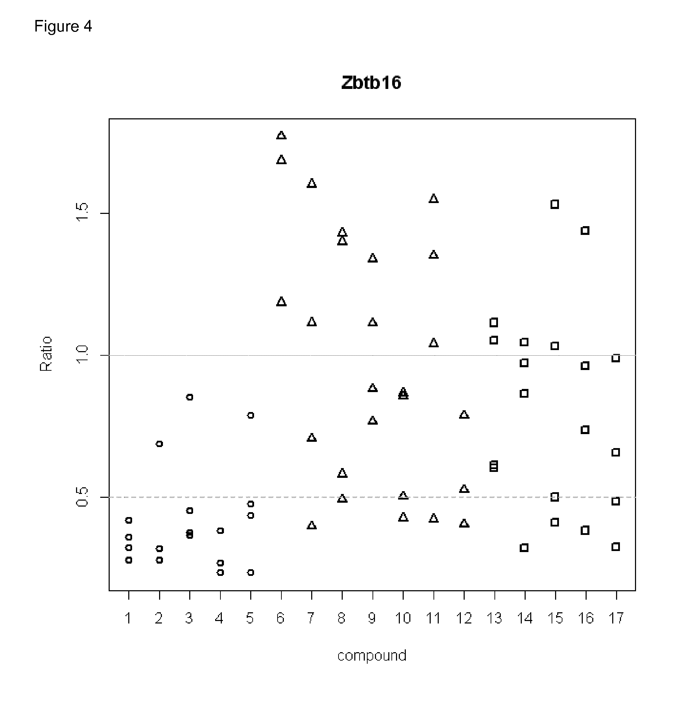

In case the method is performed with a single rodent for each compound, the results are still much better than with any prior art method available. Figure 4 shows that from the 19 individual animals exposed to 5 different GTXC compounds, the method employing marker gene *Zbtb16* would have predicted the GTXC compound right in 16/19 cases, i.e. a false negative rate of 3/19 or 16%. Even more importantly, the NGTXC compounds would have been predicted right in 21 out of 26 cases, or a false positive rate of only 5/26 or 19%. The NC compounds would have been predicted right in 14 out of 20 cases or a false positive rate of 30%.

A skilled person will understand that the method according to the invention may be performed with each single marker gene as disclosed herein and that the reliability of the method increases when 2, 3, or even all 4 marker genes are employed. Any combination of the 4 genes seemed equally suitable for the purpose.

In a method according to the invention wherein more than one gene is employed, the expression level of each individual gene tested may be used in the evaluation of the genotoxicity and or carcinogenicity of the compound. For example, a method according to the invention employing all four marker genes as described above could be described as an *in vitro* method for determining whether it is likely or unlikely that a compound is a genotoxic carcinogen wherein the expression level of marker genes *LOC75771, Nup62, Zbtb16* and *Anks4b* is determined in a sample obtained from a rodent previously exposed to the compound, wherein it is concluded that the compound is likely to be a genotoxic carcinogen if the expression level of *LOC75771* or *Anks4b* is above a predetermined reference value or if the expression level of *Nup62* or *Zbtb16* is below a predetermined reference value or concluding that the compound is unlikely a genotoxic carcinogen if the expression level of *LOC75771* or *Anks4b* is at or below a predetermined reference value or if the expression level of *Nup62* or *Zbtb16* is at or above a predetermined reference value.

In order to increase the reliability of the method and/or the specificity, the method according to the invention may be performed by determining the expression level of more than one of the marker genes as described above and concluding that the compound is likely to be a genotoxic carcinogen if any combination of 2 or more marker genes is over- or underexpressed. Conversely, the method may also be performed by determining the expression level of more than one of the marker genes as described above and concluding that the compound is unlikely to be a genotoxic carcinogen if only one marker genes is over- or underexpressed.

In order to further increase the reliability of the method and/or the specificity, the method according to the invention may also be performed by determining the expression level of more than two of the marker genes as described above and concluding that the compound is likely to be a genotoxic carcinogen if any combination of 2 or more (such as 3 or 4) marker genes are over- or underexpressed. Conversely, the method may also be performed by determining the expression level of more than two of the marker genes as described above and concluding that the compound is unlikely to be a genotoxic carcinogen if 2 or less (such as 1 or 0) as marker genes are over- or underexpressed.

In order to even further increase the reliability of the method and/or the specificity, the method according to the invention may also be performed by determining the expression level of all 4 of the marker genes as described above and concluding that the compound is likely to be a genotoxic carcinogen if at least 2 (such as 3 or 4) marker genes are over- or underexpressed. Conversely, the method may also be performed by determining the expression level of more than two of the marker genes as described above and concluding that the compound is unlikely to be a genotoxic carcinogen if 3 or less (such as 2, 1 or 0) as marker genes are over- or underexpressed.

The skilled person will know how to combine the marker genes disclosed herein in a method suited for the purpose. For example, if it is important to avoid as many risk as possible (such as for instance in the development of a medicament) then sensitivity of the method is of utmost importance. The method may then be designed in that all four marker genes are tested and the compound is discarded as a genotoxic carcinogen if at least one of the marker genes is positive, i.e. below or above the predetermined reference value.

We also found marker genes that could distinguish between NGTXC and NC compounds once it was established that a compound is not a GTXC compound. For that purpose, marker genes *9030619P08Rik* and *Cyp17a1* appeared particularly useful. Hence, the invention also relates to a method for determining whether it is likely or unlikely that a compound is a carcinogen by first performing a method as described above wherein it is concluded that a compound is unlikely to be a genotoxic carcinogen, followed by a step wherein the expression level of at least one second marker gene is determined in a sample obtained from a rodent previously exposed to the compound, wherein the at least one second marker gene is selected from the group consisting of *9030619P08Rik* and *Cyp17a1,* and wherein it is concluded that the compound is likely to be a non-carcinogenic compound if the expression level of *9030619P08Rik* and/or *Cyp17a1* is below a predetermined reference value or wherein it is concluded that the compound is likely a non-genotoxic carcinogenic compound if the expression level of *9030619P08Rik* and/or *Cyp17a1* is at or above a predetermined reference value.

It may be determined from the results shown in tables 5 and 6 that a NGTXC compound may be distinguished from a NC compound on the basis of the Fold Change of expression of marker gene *Cyp17a1.* In the experimental set-up as chosen, wherein the method was performed in 3 - 4-fold, it is most advantageous to select a predetermined reference value of 2. Under those conditions, 5 out of 5 NGTXC compounds were found positive in the method according to the invention whereas 4 out of 5 NC compounds were negative. This results in a sensitivity of 100% and a specificity of 80%.

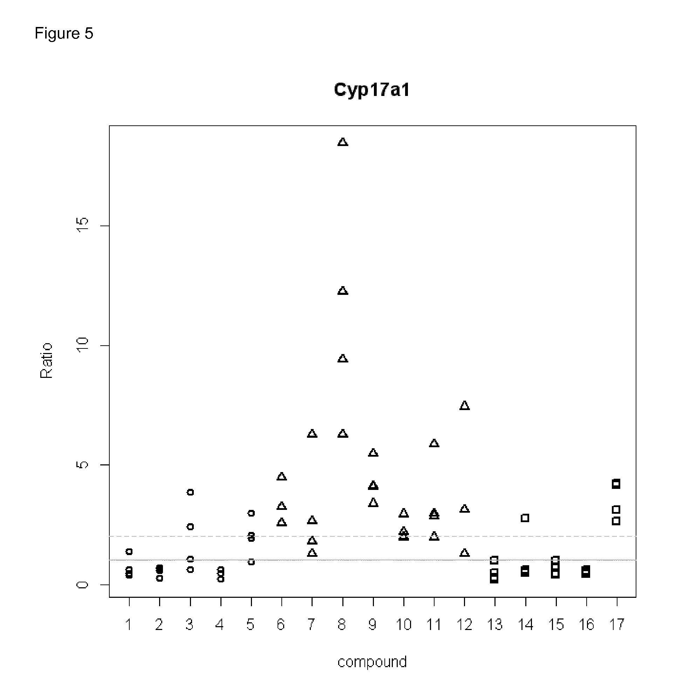

In case the method is performed with a single rodent for each compound, the results are still much better than with any prior art method available. Figure 5 shows that from the 26 individual animals exposed to 7 different NGTXC compounds, the method employing marker gene *Cyp17a1* would have predicted the NGTXC compound right in 20/26 cases, i.e. a false negative rate of 6/26 or 23%. The NC compounds would have been predicted right in 15 out of 20 cases or a false positive rate of 20%.

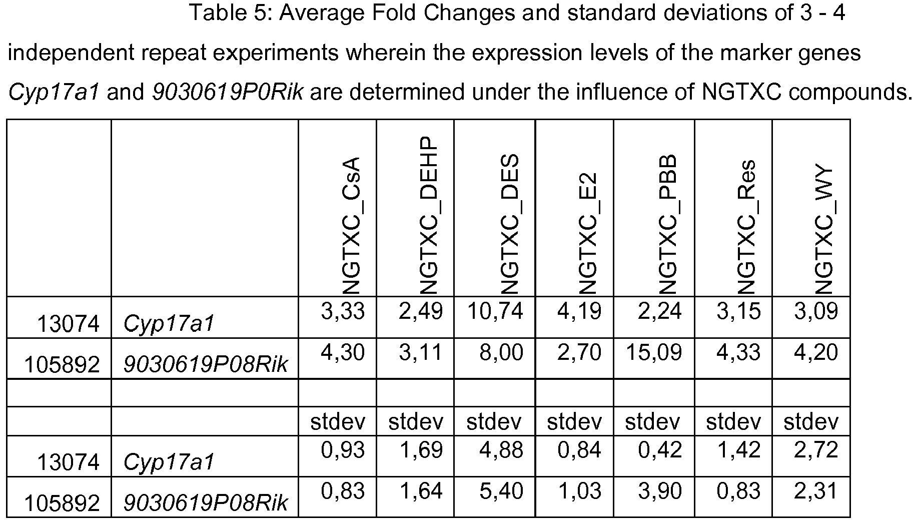

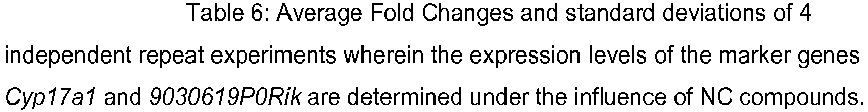

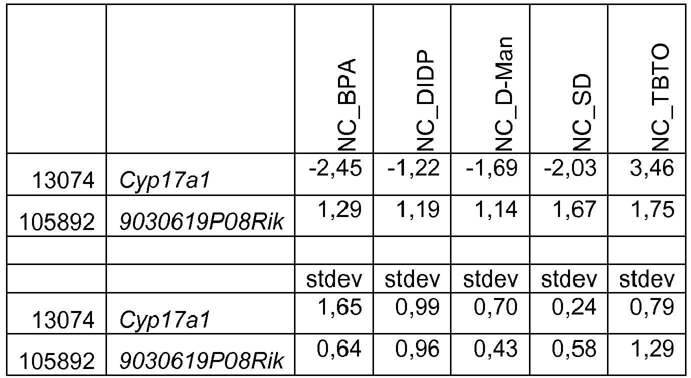

It may also be determined from the results shown in tables 5 and 6 that a NGTXC compound may be distinguished from a NC compound on the basis of the Fold Change of expression of marker gene *9030619P08Rik.* In the experimental set-up as chosen, wherein the method was performed in 3-4-fold, it was most advantageous to select a predetermined reference value of 2. Under those conditions, 5 out of 5 NGTXC compounds were found positive in the method according to the invention whereas also 5 out of 5 NC compounds were negative. This results in a sensitivity of 100% and a specificity of 100%.

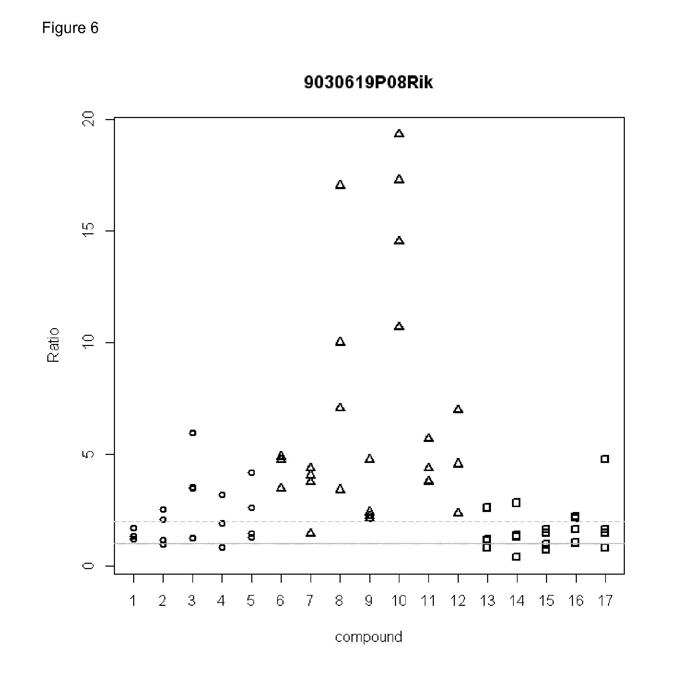

In case the method is performed with a single rodent for each compound, the results are still much better than with any prior art method available. Figure 6 shows that from the 26 individual animals exposed to 7 different NGTXC compounds, the method employing marker gene *9030619P08Rik* would have predicted the NGTXC compound right in 25/26 cases, i.e. a false negative rate of 1/26 or 4%. The NC compounds would have been predicted right in 15 out of 20 cases or a false positive rate of 25%.

It may also be concluded from the data presented herein that marker genes *9030619P08Rik* and *Cyp17a1* are suitable for distinguishing NGTXC compounds from GTXC and NC (tables 5, 6 and 7 and figures 5 and 6). This however can only be performed with an acceptable specificity when high reference values are employed, such as 5 or 6 or above, resulting in a rather poor sensitivity of the method. However, when a high specificity is required, such a method may deliver advantageous results.

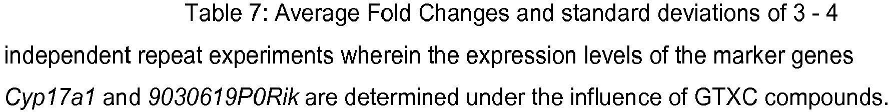

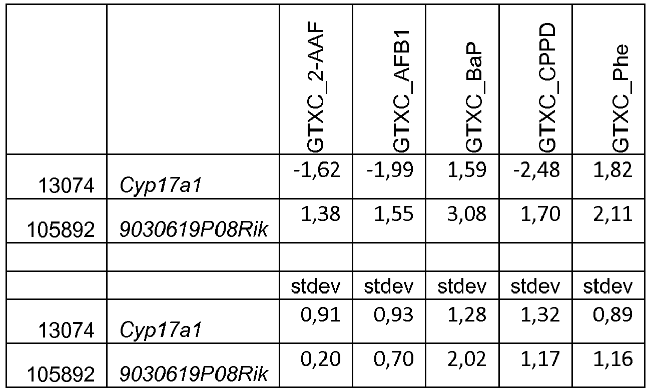

A skilled person will understand that a method according to the invention may be performed with each single marker gene as disclosed herein and that the reliability of the method increases when both marker genes are employed. This may be combined with a method for determining the genotoxic carcinogenicity of a compound as described above wherein 1, 2, 3 or all 4 marker genes are employed selected from the group consisting of *LOC75771, Nup62, Zbtb16* and *Anks4b.*

It will be apparent to the skilled person that the results presented herein may be used in many different ways in order to discriminate between carcinogenic and non-carcinogenic compounds. It will also be apparent to the skilled person that the results presented herein may be used in many different ways in order to discriminate between genotoxic and non-genotoxic compounds. As shown herein, a method according to the invention is equally suited to discriminate between NGTXC, GTXC and NC compounds, in either a one-step assay of a two step assay, depending on the desired outcome and reliability of the method. Also, methods according to the invention may employ other and additional marker genes so that the robustness, reliability, sensitivity and/or specificity of the method may be even further improved.

### Legend to the figures

**Figure 1** shows the individual expression levels of gene LOC75771 in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11 and WY in column 12 and to NC compounds BPA in column 13, DIDP in column 14, D-man in column 15, SD in column 16 and TBTO in column 17. The Ratios are shown, i.e. over-expression of genes is shown as a value above 1.0, the under-expression of genes is shown as values between 0 and 1. Dotted line indicates the predetermined reference value or cut-off value.
**Figure 2** shows the individual expression levels of gene Anks4b in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11and WY in column 12 and to NC compounds BPA in column 13, DIDP in column 14, D-man in column 15, SD in column 16 and TBTO in column 17. The Ratios are shown, i.e. over-expression of genes is shown as a value above 1.0, under-expression of genes is shown as values between 0 and 1. Dotted line indicates the predetermined reference value or cut-off value.
**Figure 3** shows the individual expression levels of gene Nup62 in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11and WY in column 12 and to NC compounds BPA in column 13, DIDP in column 14, D-man in column 15, SD in column 16 and TBTO in column 17. The Ratios are shown, i.e. over-expression of genes is shown as a value above 1.0, under-expression of genes is shown as values between 0 and 1. Dotted line indicates the predetermined reference value or cut-off value.
**Figure 4** shows the individual expression levels of gene Zbtb16 in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11and WY in column 12 and to NC compounds BPA in column 13, DIDP in column 14, D-man in column 15, SD in column 16 and TBTO in column 17. The Ratios are shown, i.e. over-expression of genes is shown as a value above 1.0, under-expression of genes is shown as values between 0 and 1. Dotted line indicates the predetermined reference value or cut-off value.
**Figure 5** shows the individual expression levels of gene Cyp17a1 in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11 and WY in column 12 and to NC compounds BPA in column 13, DIDP in column 14, D-man in column 15, SD in column 16 and TBTO in column 17. The Ratios are shown, i.e. over-expression of genes is shown as a value above 1.0, under-expression of genes is shown as values between 0 and 1. Dotted line indicates the predetermined reference value or cut-off value.
**Figure 6** shows the individual expression levels of gene 9030619P08Rik in samples obtained from 3 - 4 individual animals exposed to GTXC compounds AAF in column 1, AFB1 in column 2, BaP in column 3, CPPD in column 4 and Phe in column 5, and to NGTXC compounds CsA in column 6, DEHP in column 7, DES in column 8, E2 in column 9, PBB in column 10, Res in column 11and WY in column 12 and to NC compounds BPA in column 13, DIDP in column 14, D-man in column 15, SD in column 16 and TBTO in column 17. The Ratios are shown, i.e. over-expression of genes is shown as a value above 1.0, under-expression of genes is shown as values between 0 and 1. Dotted line indicates the predetermined reference value or cut-off value.

### Examples

### Example 1: Animals

Eight-week-old male WT mice (C57BL/6J) were acclimated for 2 weeks prior to the experiment and subsequently treated for 7 days with a compound known to be genotoxic, non-genotoxic or non-carcinogen *in vivo.* Animals were exposed to the compound through feed, gavage or intraperitoneal (i.p.) injection. The health status of the mice was monitored daily, beginning at the day of weaning. During acclimatization and the duration of the experiment, animals were kept in the same stringently controlled (specific pathogen-free, spf) environment, fed ad libitum, kept under a normal day/night rhythm and weighed weekly. After 7 days of exposure mice were sacrificed at a fixed time of the day. During autopsy liver, blood, spleen, kidney, bladder, bone marrow, lung, stomach and thymus were isolated and stored according to protocol using RNAlater (Qiagen, Valencia, CA, USA).

### Example 2: In vivo short-term exposure studies

Animals were exposed to the various compounds used in this study at the appropriate non-cytotoxic dosages. Details of the chemicals used in the studies are shown in Table 8 below. Exposure through feed was continuously and ad libitum during the experiment, application using i.p. injection was 3 times per week (day 0, 3, 6) and exposure using gavage was 4 times per week (day 0, 2, 4, 6). Comparison of different control groups (gavage, i.p. injection or feed) showed no significant differential effect on transcriptional levels.

### Example 3: RNA isolation and mRNA expression profiling.

Total RNA from mouse liver was isolated using the microRNeasy kit (Qiagen, Valencia, CA, USA) and the QIAcube (Qiagen, Valencia, CA, USA) according to the manufacturer's instructions. RNA quality was tested using capillary gel electrophoresis (Bioanalyzer 2100; Agilent Technologies, Amstelveen, the Netherlands). Transcriptomic analysis was performed by microarray analysis (Affymetrix). Individual expression levels of each marker gene in each animal are shown in table 9.

**Table 8 Full overview of compounds used and their details.**

| **Chemical** | **Abbreviation** | **Class** | **CAS No.** | **Dose** | **Administration** |
|---|---|---|---|---|---|
| Control | - | - | - | - | Feed |
| 2-Acetylaminofluorene | 2-AAF | GTXC | 53-96-3 | 300 ppm | Feed |
| Aflatoxin B1 | AFB1 | GTXC | 1162-65-8 | 1 ppm | Feed |
| Benzo(a)pyrene | BaP | GTXC | 50-32-8 | 13 mg/kg bw | Gavage^{*} |
| Cisplatin | CPPD | GTXC | 15663-27-1 | 0.6 mg/kg bw | i.p. injection^{#} |
| Phenacetin | Phe | GTXC | 62-44-2 | 7500 ppm | Feed |
| 17 β- Estradiol | E2 | NGTXC | 50-28-2 | 5 mg/kg bw | Gavage^{†} |
| Cyclosporin A | CsA | NGTXC | 59865-13-3 | 500 ppm | Feed |
| Di(2-ethylhexyl)phthalate | DEHP | NGTXC | 117-81-7 | 6000 ppm | Feed |
| Diethylstillbestrol | DES | NGTXC | 56-53-1 | 1.5 ppm | Feed |
| Phenobarbital | PBB | NGTXC | 57-30-7 | 1500 ppm | Feed |
| Reserpine | Res | NGTXC | 50-55-5 | 5 ppm | Feed |
| Wyeth-14643 | WY | NGTXC | 50892-23-4 | 250 ppm | Feed |
| Bisphenol A | BPA | NC | 80-05-7 | 5000 ppm | Feed |
| Diisodecyl phthalate | DIDP | NC | 26761-40-0 | 2500 ppm | Feed |
| D-Mannitol | D-man | NC | 69-65-8 | 50.000 ppm | Feed |
| Sodium diclofenac | SD | NC | 15307-79-6 | 25 ppm | Feed |
| Tributyl-tin-oxide | TBTO | NC | 56-35-9 | 200 ppm | Feed |

| | | | | | |
|---|---|---|---|---|---|
| Solvent: * = sunflower oil, # = PBS, t = methyl cellulose | | | | | |

**Table 9: Fold Change of expression of different genes in individual animals tested under the influence of different compounds as indicated.**

| **Exposure** | **Fig** | **LOC75771** | **Anks4b** | **Nup62** | **Zbtb16** | | **Cyp17a1** | **9030619P08Rik** |
|---|---|---|---|---|---|---|---|---|
| GTXC_2-AAF_682 | 1 | 1,705921 | 1,655817 | -1,55724 | -3,61818 | | 1,364397 | 1,187438 |
| GTXC_2-AAF_683 | 1 | 2,011494 | 2,059207 | -1,69925 | -2,77847 | | -2,15504 | 1,340597 |
| GTXC_2-AAF_684 | 1 | 1,050507 | 2,270806 | -1,3329 | -3,11926 | | -2,61584 | 1,335409 |
| GTXC_2-AAF_685 | 1 | 1,984839 | 1,587469 | -1,46527 | -2,3778 | | -1,66057 | 1,684509 |
| GTXC_AFB1_657 | 2 | 1,417281 | 1,858186 | -1,73057 | -3,60257 | | -3,98454 | -1,03893 |
| GTXC_AFB1_658 | 2 | 1,802676 | 2,278459 | -1,73075 | -3,13223 | | -1,45328 | 2,509845 |
| GTXC_AFB1_659 | 2 | 2,122773 | 1,940009 | -1,41994 | -3,14589 | | -1,5429 | 2,053756 |
| GTXC_AFB1_660 | 2 | 1,042438 | 1,643602 | -1,39121 | -1,45081 | | -1,75477 | 1,171552 |
| GTXC_BaP_591 | 3 | 1,573528 | 1,590135 | -1,41015 | -1,1713 | | 3,849942 | 5,940256 |
| GTXC_BaP_592 | 3 | 2,511058 | 2,091694 | -1,46302 | -2,2075 | | 1,069143 | 3,463285 |
| GTXC_BaP_593 | 3 | 2,153342 | 1,512848 | -1,51176 | -2,73557 | | -1,57076 | 3,508919 |
| GTXC_BaP_594 | 3 | 1,654892 | 1,249576 | -1,24257 | -2,65608 | | 2,426957 | 1,242329 |
| GTXC_CPPD_753 | 4 | 2,205733 | 1,894611 | -1,40437 | -4,28606 | | -4,49668 | 1,882632 |
| GTXC_CPPD_754 | 4 | 1,720149 | 1,416897 | -1,37437 | -2,61037 | | -2,09626 | 3,19679 |
| GTXC_CPPD_756 | 4 | 2,049812 | 1,570269 | -1,38035 | -3,7119 | | -1,61055 | -1,23124 |
| GTXC_Phe_520 | 5 | 1,462697 | 1,592469 | -1,46978 | -4,26686 | | 1,929473 | 2,591988 |
| GTXC_Phe_521 | 5 | 1,408953 | 1,221871 | -1,41748 | -1,26966 | | -1,08575 | 1,278319 |
| GTXC_Phe_522 | 5 | 1,858825 | 1,552723 | -1,3917 | -2,10594 | | 2,976794 | 1,436254 |
| GTXC_Phe_523 | 5 | 1,503018 | 1,512243 | -1,33382 | -2,30082 | | 2,054986 | 4,17095 |
| NGTXC_CsA_621 | 6 | 1,079978 | 1,071466 | -1,4609 | 1,686399 | | 2,560019 | 4,874438 |
| NGTXC_CsA_622 | 6 | 1,022067 | 1,254999 | -1,03782 | 1,185501 | | 4,4578 | 4,738075 |
| NGTXC_CsA_623 | 6 | 1,1929 | 1,324918 | -1,14146 | 1,771223 | | 3,244853 | 3,448918 |
| NGTXC_DEHP_557 | 7 | 1,152676 | -1,08 | 1,166832 | -1,41583 | | 6,253363 | 1,421539 |
| NGTXC_DEHP_558 | 7 | 1,339443 | -1,18919 | 1,023361 | 1,601817 | | 1,806164 | 3,741746 |
| NGTXC_DEHP_559 | 7 | 1,013056 | 1,270926 | -1,1264 | -2,52071 | | 1,292719 | 4,049144 |
| NGTXC_DEHP_560 | 7 | 1,023425 | -1,59617 | 1,077398 | 1,114567 | | 2,626167 | 4,353386 |
| NGTXC_DES_533 | 8 | -1,13239 | 1,020605 | -1,56356 | -1,72326 | | 9,417864 | 3,399244 |
| NGTXC_DES_534 | 8 | -1,07708 | 1,193296 | 1,18178 | 1,430939 | | 18,44632 | 17,02687 |
| NGTXC_DES_535 | 8 | 1,027336 | -1,09903 | -1,04186 | 1,400903 | | 6,256012 | 10,00524 |
| NGTXC_DES_536 | 8 | -1,04436 | -1,20697 | 1,041463 | -2,03597 | | 12,22566 | 7,062258 |
| NGTXC_E2_539 | 9 | -1,20805 | -1,80496 | 1,098928 | -1,13449 | | 4,0752 | 2,406869 |
| NGTXC_E2_540 | 9 | 1,023059 | -1,16666 | -1,10136 | 1,33934 | | 4,110967 | 4,751935 |
| NGTXC_E2_541 | 9 | -1,00513 | 1,006628 | -1,23304 | -1,30415 | | 3,369521 | 2,101086 |
| NGTXC_E2_542 | 9 | -1,20816 | -1,06793 | -1,14583 | 1,112228 | | 5,471111 | 2,208578 |
| NGTXC_PBB_543 | 10 | 1,084573 | 1,857325 | -1,18737 | -2,34418 | | 1,987088 | 10,68519 |
| NGTXC_PBB_544 | 10 | 1,085274 | 1,468237 | -1,21348 | -1,15391 | | 1,966993 | 17,28227 |
| NGTXC_PBB_545 | 10 | 1,34375 | 1,631778 | 1,070337 | -1,99396 | | 2,933095 | 19,31769 |
| NGTXC_PBB_546 | 10 | 1,18945 | 1,048775 | 1,356669 | -1,17093 | | 2,191937 | 14,53071 |
| NGTXC_Res_551 | 11 | 1,258758 | 1,249403 | -1,45012 | 1,041437 | | 5,862861 | 5,675164 |
| NGTXC_Res_552 | 11 | 1,891296 | 1,659617 | -1,50141 | -2,3621 | | 1,9799 | 3,768901 |
| NGTXC_Res_553 | 11 | -1,26214 | -1,2468 | -1,0185 | 1,548637 | | 2,951442 | 3,774088 |
| NGTXC_Res_554 | 11 | 1,026105 | -1,1247 | -1,04124 | 1,351465 | | 2,859325 | 4,359835 |
| NGTXC_WY_628 | 12 | 1,423422 | -1,07018 | 1,009725 | -1,90737 | | 7,432952 | 2,341437 |
| NGTXC_WY_629 | 12 | 1,516343 | 1,103893 | -1,03582 | -2,47386 | | 1,276397 | 4,552929 |
| NGTXC_WY_630 | 12 | -1,05136 | 1,070127 | 1,080088 | -1,27039 | | 3,103282 | 6,965959 |
| NC_BPA_663 | 13 | 1,027932 | -1,16229 | -1,01361 | 1,114306 | | -4,75677 | -1,23514 |
| NC_BPA_664 | 13 | 1,306211 | 1,285365 | -1,00973 | -1,6599 | | -1,01075 | 2,605592 |
| NC_BPA_665 | 13 | -1,1049 | -1,14224 | 1,14385 | 1,05274 | | -3,4994 | 1,167664 |
| NC_BPA_666 | 13 | 1,370273 | -1,06695 | -1,3266 | -1,63459 | | -2,12839 | 1,121763 |
| NC_DIDP_669 | 14 | 1,468884 | 1,254408 | 1,082226 | 1,045284 | | -1,67941 | 1,367458 |
| NC_DIDP_670 | 14 | 1,270453 | -1,44863 | -1,02869 | -1,02838 | | 2,746697 | -2,48269 |
| NC_DIDP_671 | 14 | 1,155297 | 1,229508 | 1,206993 | -1,15546 | | -2,01664 | 1,316609 |
| NC_DIDP_672 | 14 | 1,177232 | 1,74936 | -1,44589 | -3,11558 | | -1,81914 | 2,807593 |
| NC_D-Man_561 | 15 | 1,08954 | 1,041953 | 1,051429 | 1,033201 | | -1,02234 | -1,37027 |
| NC_D-Man_562 | 15 | 1,108244 | -1,27977 | -1,05706 | 1,530829 | | -2,54228 | 1,478092 |
| NC_D-Man_563 | 15 | 1,368446 | 1,795032 | -1,22113 | -2,43981 | | -2,18355 | -1,05581 |
| NC_D-Man_564 | 15 | 1,430951 | 1,387402 | -1,1502 | -2,00161 | | -1,43774 | 1,644727 |
| NC_SD_675 | 16 | 1,026292 | 1,138238 | -1,0507 | 1,43991 | | -2,24175 | 1,030832 |
| NC_SD_676 | 16 | 1,184061 | 1,116068 | -1,25371 | -1,35914 | | -1,72766 | 2,114699 |
| NC_SD_677 | 16 | 1,388857 | 1,354291 | -1,38913 | -2,61522 | | -2,19308 | 1,629067 |
| NC_SD_678 | 16 | 1,675938 | 1,445565 | -1,05708 | -1,04012 | | -2,01351 | 2,206369 |
| NC_TBTO_651 | 17 | 1,117869 | 1,512535 | -1,27956 | -3,09643 | | 4,236106 | 1,48253 |
| NC_TBTO_652 | 17 | 2,017137 | 1,533457 | -1,17819 | -1,01145 | | 4,133365 | 1,633938 |
| NC_TBTO_653 | 17 | 1,50697 | 1,374099 | -1,32942 | -1,52353 | | 3,102983 | -1,22599 |
| NC_TBTO_654 | 17 | 1,210468 | 1,172742 | -1,04656 | -2,06942 | | 2,636019 | 4,776732 |

## Claims

1. *In vitro* method for determining whether it is likely or unlikely that a compound is a genotoxic carcinogen wherein the expression level of at least one marker gene is determined in a sample obtained from a rodent previously exposed to the compound, wherein the at least one marker gene is selected from the group consisting of *LOC75771, Nup62, Zbtb16* and *Anks4b,* and wherein it is concluded that the compound is likely to be a genotoxic carcinogen if the expression level of *LOC75771* or *Anks4b* is above a predetermined reference value or if the expression level of *Nup62* or *Zbtb16* is below a predetermined reference value or concluding that the compound is unlikely a genotoxic carcinogen if the expression level of *LOC75771* or *Anks4b* is at or below a predetermined reference value or if the expression level of *Nup62* or *Zbtb16* is at or above a predetermined reference value.

2. Method according to claim 1 wherein the at least one marker gene is at least two marker genes, such as at least 3 marker genes such as at least 4 marker genes.

3. Method according to claims 1 - 2 wherein it is concluded that the compound is likely to be a genotoxic carcinogen if the Ratio of *LOC75771* or *Anks4b* is above 1.5, or if the Ratio of *Nup62* is below 0.75 (Fold Change < -1.33) or if the Ratio of *Zbtb16* is below 0.5 (Fold Change < -2) or concluding that the compound is unlikely to be a genotoxic carcinogen if the Ratio of *LOC75771* or *Anks4b* is at or below 1.5, or if the Ratio of *Nup62* is at or above 0.75 or if the Ratio of *Zbtb16* is at or above 0.5.

4. Method for determining whether it is likely or unlikely that a compound is a carcinogen by first performing a method according to any one of claims 1 - 3 and concluding that a compound is unlikely a genotoxic carcinogen, followed by a step wherein the expression level of at least one second marker gene is determined in a sample obtained from a rodent previously exposed to the compound, wherein the at least one second marker gene is selected from the group consisting of *9030619P08Rik* and *Cyp17a1,* and wherein it is concluded that the compound is likely to be a non-carcinogenic compound if the expression level of *9030619P08Rik* and/or *Cyp17a1* is below a predetermined reference value or wherein it is concluded that the compound is likely a non-genotoxic carcinogenic compound if the expression level of *9030619P08Rik* and/or *Cyp17a1* is at or above a predetermined reference value.

5. Method according to claim 4 wherein the at least one second marker gene is at least two second marker genes.

6. Method according to claims 4 or 5 wherein it is concluded that the compound is likely a non-carcinogenic compound if the Ratio of *9030619P08Rik* or *Cyp17a1* is below 2.0.

7. Method according to claims 4 or 5 wherein it is concluded that the compound is unlikely to be a non-carcinogenic compound if the Ratio of *9030619P08Rik* or *Cyp17a1* is at or above 2.0.

8. Method according to claims 1 - 7 wherein the sample is obtained from an organ or a tissue selected from the group consisting of liver, blood, spleen, kidney, bladder, bone marrow, lung, stomach and thymus.

9. Method according to claims 1 - 8 wherein the rodent is exposed to the sample for at least 1 day such as 3 days or one week.

10. Method according to claims 1 - 9 wherein the rodent is a mouse or a rat, preferably an inbred strain laboratory mouse or rat.

11. Method according to claim 10 wherein the mouse is a C57 Black mouse, preferably a C57BLC6 mouse, more preferred a C57BL/6J mouse.

12. Method according to claims 1 - 11 wherein the predetermined reference value is at least 1.25 times such as 1.50 or 1.75 or even at least 2 times the control expression level of the same marker gene in a sample from an animal not exposed to the compound.

13. Method according to claims 1 - 11 wherein the predetermined reference value is a value of at least the expression level of the same marker gene in a sample from an animal not exposed to the compound divided by at least 1.25 such as 1.50 or 1.75 or even at least 2.

14. Method according to claims 1 - 13 wherein multiple individual rodents are exposed to the same compound and wherein the expression level is the average of the individual expression levels of each individual gene.

15. Method according to claim 14 wherein 3 or more rodents such as 4 or more rodents, such as 5, 6, 7, 8, 9 or 10 or more are used per compound.
